# EUROPÄISCHE PATENTSCHRIFT

(11) **EP 2 001 366 B1**
(45) Veröffentlichungstag und Bekanntmachung des Hinweises auf die Patenterteilung: **06.05.2015**
(21) Anmeldenummer: 07701908.1
(22) Anmeldetag: 02.03.2007
(51) Int. Cl.: A61B 6/04

(54) **VERFAHREN UND VORRICHTUNG ZUM HEBEN UND SENKEN SOWIE DREHEN UND HORIZONTALEN VERFAHREN EINER PATIENTENLIEGE**
METHOD AND DEVICE FOR LIFTING, LOWERING, ROTATING AND HORIZONTALLY MOVING A PATIENT SUPPORT
PROCEDE ET DISPOSITF DE RELEVEMENT, D'ABAISSEMENT AINSI QUE DE ROTATION ET DE DEPLACEMENT HORIZONTAL D'UNE COUCHETTE POUR ÜPATIENT

(30) Priorität: 31.03.2006 CH 5372006
(43) Veröffentlichungstag der Anmeldung: 17.12.2008
(73) Patentinhaber: Schär Engineering AG, 8416 Flaach (CH)
(72) Erfinder: SCHÄR, Hugo, CH-8412 Aesch (CH); BÄCHTOLD, David, CH-8462 Rheinau (CH)
(74) Vertreter: Gachnang, Hans Rudolf
(86) Internationale Anmeldenummer: PCT/CH2007/000108
(87) Internationale Veröffentlichungsnummer: WO 2007/112602

(56) Entgegenhaltungen:
- DE-A1-102004 041 897
- DE-B- 1 061 032
- US-A- 6 094 760

## Beschreibung

Gegenstand der Erfindung ist Verfahren und eine Vorrichtung zum Heben und Senken sowie Drehen und horizontalen Verfahren einer Patientenliege gemäss Oberbegriff der Patentansprüche 1 und 5.

In Bestrahlungsanlagen, wie beispielsweise Photonen- und Protonenbestrahlungsanlagen zur Behandlung von Tumoren, muss die Patientenliege mit dem darauf liegenden Patienten in allen Dimensionen mit einer Genauigkeit von 0,2-0,5 mm positioniert werden können, um den Energiestrahl exakt auf den zuvor andernorts oder zu anderer Zeit röntgentechnisch lokalisierten Tumor richten zu können. Für die exakte Ausrichtung des Energiestrahls sind entsprechende Vorrichtungen bekannt und es kann davon ausgegangen werden, dass der Energiestrahl in allen drei Dimensionen genau ausgerichtet werden kann. Probleme ergeben sich jedoch bei der Patientenliege, d.h. dem verfahr- und drehbaren sowie in der Höhe verstellbaren Auflagetisch, auf dem der Patient liegt und damit exakt unter der Bestrahlungsanlage positioniert wird. Die Durchbiegungen der für die Ausrichtung der Patientenliege in allen Dimensionen notwendigen Elemente sowie der Patientenliege selbst lassen sich errechnen oder vorab durch Messreihen bestimmen, speichern und entsprechend durch eine Höhenjustierung der Auflager korrigieren. Eine nicht vorbestimmbare Variable ist hingegen stets der Patient, der zum Beispiel zwischen 20 kg und 150 kg wiegen kann. Zudem ist nebst dem Gewicht des Patienten auch der genaue Ort von dessen Schwerpunkt nicht berechenbar, weshalb nicht allein aufgrund des Gewichts des Patienten entsprechende Korrekturen zur Kompensation der Durchbiegungen genügen.

Aus der US-6,094,760 ist eine Patientenliege bekannt, die in der Horizontalen schwenk- und verschiebbar sowie in der Höhe verstellbar ist. Die durch die unterschiedliche Lastverteilung je nach Grösse und Gewicht des Patienten hervorgerufene Durchbiegung kann in dieser bekannten Vorrichtung nicht erfolgen.

Eine Aufgabe der vorliegenden Erfindung besteht darin, ein Verfahren zu schaffen, mit dem die Lastverteilung des Patienten auf der Patientenliege erfasst und für die Kompensation der Durchbiegung verarbeitet werden kann.

Eine weitere Aufgabe der vorliegenden Erfindung besteht nun darin, eine Vorrichtung der eingangs erwähnten Gattung derart weiterzubilden, damit die durch das Gewicht des Patienten verursachten Abweichungen der röntgendiagnostisch ermittelten Position vom Sollwert infolge Durchbiegungen der Patientenliege ausgleichen zu können.

Gelöst wird diese Aufgabe durch ein Verfahren und eine Vorrichtung mit den Merkmalen der Patentansprüche 1 und 5. Vorteilhafte Ausgestaltungen der Erfindung sind in den abhängigen Ansprüchen definiert.

Es gelingt mit dem erfindungsgemässen Verfahren und der erfindungsgemässen Vorrichtung, den Patienten in unterschiedliche Therapiepositionen zu verfahren, ohne die Position mit Röntgenbildern überprüfen zu müssen, da die Durchbiegungen automatisch korrigiert werden können.

Anhand eines illustrierten Ausführungsbeispiels wird die Erfindung näher erläutert. Es zeigen
- Figur 1: eine perspektivische Ansicht einer Patientenliege in der Grundstellung,
- Figur 2: eine perspektivische Darstellung der Patientenliege mit teilweise ausgefahrener und geschwenkter Position,
- Figur 3: eine Seitenansicht der Hubvorrichtung für die erfindungsgemässen Patientenliege in der Grundstellung gemäss Figur 1,
- Figur 4: eine Seitenansicht der Patientenliege in ausgefahrener Stellung, Patientenliege zudem ausgeschwenkt wie in Figur 2 ersichtlich,
- Figur 5: eine Seitenansicht der Patientenliege in ausgefahrener Stellung, Patientenliege zudem ausgeschwenkte und unter Last wie in Figur 2 ersichtlich.

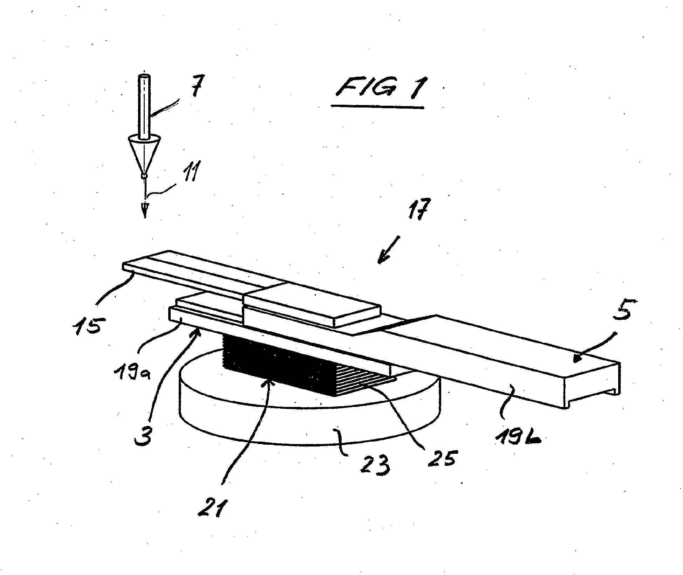

In Figur 1 ist in perspektivischer Darstellung eine Patientenliege 15, welche an einem Patiententisch 17 dreh-, schwenk- und vertikal verfahrbar befestigt ist, dargestellt. Die Patientenliege 15 ist ohne Gantry, d.h. ohne eine Vorrichtung, welche die Bestrahlungsanlage trägt und in Arbeitsstellung fährt, dargestellt. Eine Bestrahlungsanlage ist mit Bezugszeichen 7 schematisch dargestellt. Die Bestrahlungsanlage 7 wird in an sich bekannter Weise beispielsweise auf einer bogenförmig verlaufenden Bahn geführt, um den Eintrittswinkel des Energiestrahls exakt auf die zu bestrahlende Stelle im Körper eines Patienten 13 ausrichten zu können.

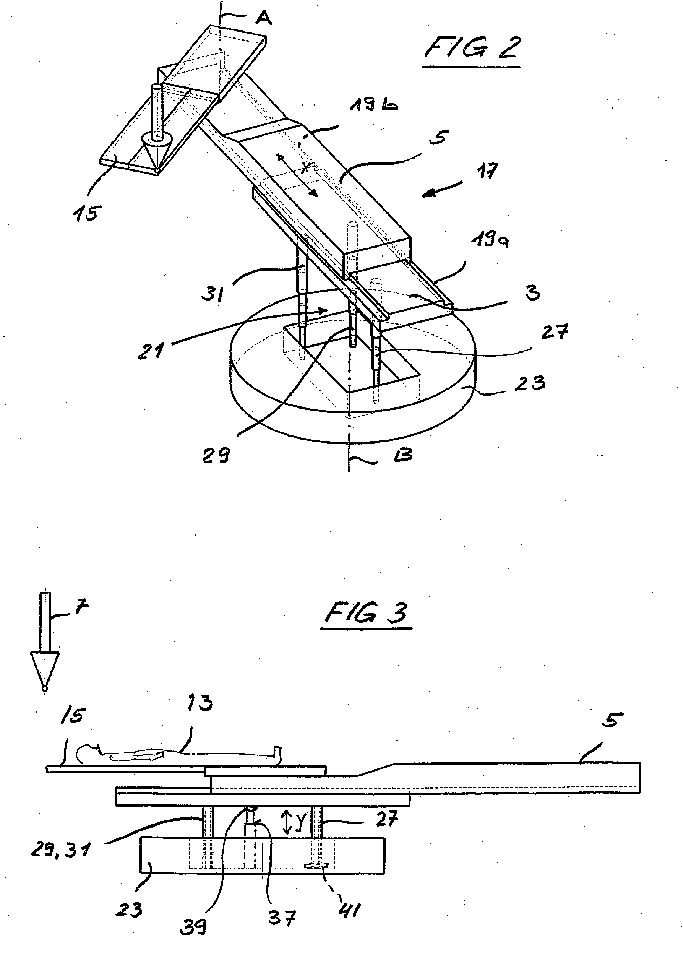

In Figur 2 ist der Patiententisch 17 mit ausgeschwenkter Patientenliege 15 wiedergegeben und wird nachfolgend beschrieben. Der Patiententisch 17 umfasst Längsführungsschienen 19a und 19b, welche auf Führungsplatten 3 und 5 angeordnet sind. Die Längsführungsplatte 3 wird an ihrer Unterseite von drei Hubspindeln 27, 29 und 31 getragen. Die drei Hubspindeln 27,29,31 sind ihrerseits auf einem Drehteller 23 gelagert. Das Auflager des Drehtellers 23, z.B. auf dem Raumboden, ist der besseren Übersichtlichkeit halber nicht dargestellt. Die drei Hubspindeln 27,29,31 bilden zusammen eine Hubvorrichtung 21, welche einerseits die vertikale Verschiebung der Patientenliege 15 ermöglicht und andererseits dienen die Hubspindeln 27,29,31, wie später beschrieben wird, auch zum Korrigieren von Seitenneigungen/Torsion der Elemente der Patientenliege 15.

Die Patientenliege 15 ist, wie beschrieben, um die vertikale Achse A schwenkbar mit dem freien Ende der Längsführungsschiene 19b verbunden. Die Längsführungsplatte 5 ist auf der Hubvorrichtung 21 in Richtung der Pfeile X linear verschiebbar gelagert. Die Dreh- und Schwenkbewegungen des Drehtellers 23 bzw. der Patientenliege 15 erfolgen mit geeignet ausgebildeten Elektroantrieben, welche nicht sichtbar sind. Ebenso erfolgt der Längshub der Längsführungsplatte 5 bezüglich der Führungsplatte 3 durch einen geeignet ausgebildeten Linearantrieb. Die entsprechenden Antriebe sind der besseren Übersichtlichkeit halber ebenfalls nicht dargestellt.

Von der Hubvorrichtung 21 ist in Figur 1 nur ein rechteckiger Balg 25 sichtbar, welcher die drei auf dem Drehteller 23 angeordneten Hubspindeln 27,29,31 vor Verschmutzung schützt. Weiter kann im Zentrum der drei in einem Dreieck angeordneten Hubspindeln 27,29,31 eine Vertikalführung 33, zum Beispiel eine Teleskop-Säule 37, angeordnet sein (in Figur 3 schematisch dargestellt). Die Säule 37 ist an ihrem unteren Ende fest mit dem Drehteller 23 verbunden. Das obere Ende der Säule 37 ist über ein Kreuzgelenk 39 oder dergleichen mit der Längsführungsplatte 3 verbunden. Die drei Hubspindeln 27, 29, 31 sind durch geeignet ausgebildete Antriebe (z.B. hydraulisch oder elektrisch) längenverstellbar, um einerseits die Patientenliege 15 in Richtung der Pfeile Y anzuheben oder abzusenken und zudem sind die Antriebe der Hubspindeln 27,29,31 einzeln ansteuerbar, um die Patientenliege 15, wenn sich diese durch das Gewicht eines Patienten 13 durchbiegt, in die horizontale Soll-Position justieren zu können. Um die Durchbiegung errechnen zu können, ist mindestens am Fuss einer der Hubspindeln, z.B. der Hubspindel 27, eine Lastmesszelle 41 eingesetzt. Es können zusätzlich bei einer oder den beiden andern Hubspindeln 29,31 Lastmesszellen 41 eingesetzt sein. Im ersten Ausführungsbeispiel, das nachfolgend beschrieben wird, steht nur eine der Hubspindeln 27 auf einer Lastmesszelle 41.

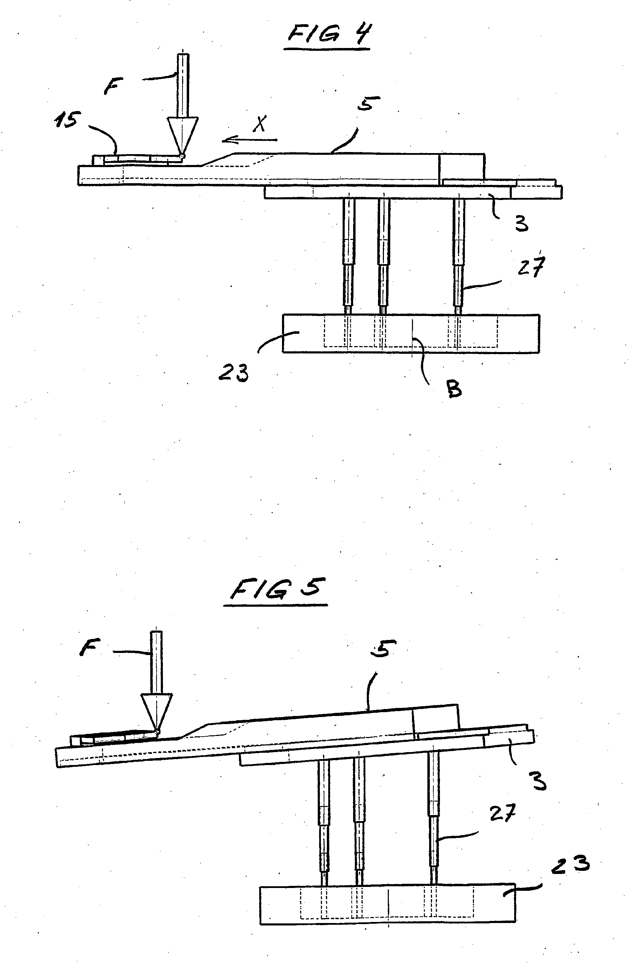

Mit den vorgängig beschriebenen Mitteln kann nebst dem Gewicht des Patienten 13 auch die Gewichtsverteilung, d.h. der Schwerpunkt des Patienten 13 auf der Patientenliege 15 bestimmt werden. Dazu sind folgende Verfahrensschritte notwendig: Die Patientenliege 15 liegt parallel zu den beiden Längsführungsschienen 19a und 19b, welche vorzugsweise zusammengefahren über der Längsführungsplatte 3 auf der Hubvorrichtung 21 angeordnet sind, wenn die Messung beginnt (Figur 3). Durch Ausfahren der Längsführungsplatte 5 aus der zusammengefahrenen übereinander liegenden Position in eine ausgefahrene Position verändert sich die Kraft auf die mit einer Lastmesszelle 41 versehene Hubspindel 27 (Figuren 4/5). Die Veränderung der Kraft über den Fahrweg in Richtung X wird in'einer Anlagensteuerung aufgezeichnet. Durch Vergleich mit einer mit einem bekannten Gewicht durchgeführten und gespeicherten Messreihe kann sowohl das Gewicht des Patienten 13 als auch die Position des Schwerpunktes des Patienten 13 festgestellt werden. Eine vorgängige Messung des Körpergewichts ist folglich nicht notwendig.

Durch die Verschiebung der Patientenliege 15 weg von der Schwenkachse B der Hubvorrichtung 21 ergibt sich eine unvermeidbare Durchbiegung einerseits der Längsführungsplatten 3,5 und andererseits der Patientenliege 15. Die Werte der Durchbiegungen der genannten Elemente können im voraus anhand von Testreihen oder empirisch ermittelt werden, so dass ein Vergleich zwischen den gemessenen Werten, d.h. die Änderung der auf die Hubspindel 27 wirkenden Kraft beim Ausfahren der Patientenliege 15 möglich ist. Ebenfalls durch Messreihen kann im voraus die notwendige Korrektur, d.h. das Ausfahren der Hubspindel 27 und/oder der Hubspindeln 29,31 für jeden gemessenen Wert gespeichert und später für das Neuausrichten der Patientenliege 15 mit einem darauf liegenden Patienten 13 erfolgen.

Das beschriebene Vorgehen beschränkte sich bis jetzt auf das Ausfahren der Längsführungsplatte 5 und der mit dieser in Linie liegenden Patientenliege 15.

In der Praxis wird die Patientenliege 15 aber zusätzlich um die Drehachse A verschwenkt, um den Patienten 13 bzw. den im Patienten 13 vorhandenen Tumor an die vorbestimmte Stelle im Strahl 11 der Bestrahlungsanlage 7 zu bringen. Beim Verschwenken der Patientenliege 5 wirken auf die beiden Längsführungsplatten 3,5 zusätzlich Torsionskräfte, welche zu einer weiteren überlagerten Verbiegung der beiden Elemente führen. Zusätzlich zu dieser durch die Torsion bewirkte Absenkung der Patientenliege 15 kommt deren eigene Durchbiegung hinzu. Wie beim erstgenannten Beispiel bei einem linearen Verschieben der Patientenliege 15 kann auch beim Schwenken der Patientenliege 15 der Kräfteverlauf an der Hubspindel 27 über den Verschwenkweg bzw. Verschwenkwinkel erfasst und mit einem zuvor in einer Messreihe festgehaltenen Wertetabelle verglichen und entsprechend die Höhenkorrektur eingeleitet werden. Die Höhenkorrektur erfolgt in diesem Fall nicht ausschliesslich mit der Hubspindel 27, welche auf einer Lastmesszelle 41 abgestützt ist, sondern zusätzlich durch mindestens eine, vorzugsweise beide andern Hubspindeln 29 oder 31, welche einerseits die Verbiegung und dadurch Abweichung der Liege 15 vom Sollwert korrigiert, anderseits wird auch die Durchbiegung der Patientenliege 15 selbst durch eine oder beide Hubspindeln 29 oder 31 korrigiert. Die Korrekturwerte, d.h. die Längenänderungen der Hubspindeln 27 bis 31 für die Korrektur der Durchbiegung, können den gespeicherten, aus Messreihen erlangten Werten entnommen werden.

In einer weiteren Ausgestaltung der Erfindung können auch zwei Hubspindeln, z.B. die Hubspindel 27 und die Hubspindel 29 auf einer Lastmesszelle 41 abgestützt sein und es kann auf diese Weise, wiederum aus gespeicherten Werten einer Messreihe die Durchbiegung der Vorrichtung bzw. deren einzelner Elemente bestimmt und die entsprechenden Korrekturwerte (Höhenverstellung der beiden Hubspindeln oder aller drei Hubspindeln) vorgenommen werden. Falls alle Hubspindeln 27,29,31 auf Lastmesszellen 41 abgestützt sind, kann die Durchbiegung der Patientenliege bzw. die örtliche Veränderung des vorbestimmten Bestrahlungspunktes im Patienten 13 ohne vorangehende Verschiebung und Messen der Änderung der Last auf den Hubspindeln 27,29,31 erfolgen. Die Hubkorrektur kann dann durch Vergleich mit gespeicherten Messreihen bezüglich der Laständerungen auf allen drei Hubspindeln 27,29,31 beim Verfahren und Verschwenken der Patientenliege 15 errechnet werden.

## Patentansprüche

1. Verfahren zum Messen und Kompensieren der von der Last eines Patienten bewirkten Durchbiegungen der Bauteile eines Patiententisches (17), auf dem der Patient (13), auf einer Patientenliege (15) liegend, in den Fokus des Energiestrahls (11) einer Bestrahlungsanlage (7) positionierbar ist, **gekennzeichnet durch** folgende Verfahrensschritte:
- Messen der Änderungen der Last bei linearem Verfahren des Patiententisches (17) an mindestens einer von drei die Patientenliege (15) tragenden Hubspindeln (27,29,31) und Korrektur/Kompensation der **durch** die jeweilige Gewichtsverteilung des Patienten hervorgerufenen Durchbiegungen der Bauteile des Patiententisches (17) **durch** Verstellen der Höhe mindestens einer der Hubspindeln (27,29,31) zur Kompensation der Durchbiegungen.

2. Verfahren nach Anspruch 1, **dadurch gekennzeichnet, dass** die Korrektur-/Kompensationswerte anhand gespeicherter, durch eine Messreihe ermittelter oder errechneter und gespeicherter Messwerte erfolgt.

3. Verfahren nach Anspruch 1, **dadurch gekennzeichnet, dass** die Korrektur-/Kompensationswerte durch
- Verschieben der Patientenliege (15) entlang einer ersten Strecke und Messen der Änderung der Last in Abhängigkeit des zurückgelegten Weges an mindestens einer von drei die Patientenliege (15) tragenden Hubspindeln (27, 29, 31),
- Vergleich des oder der an mindestens einer der Hubspindeln (27,29,31) gemessenen Lastwerte mit zuvor errechneten oder ausgemessenen, durch eine Messreihe ermittelten oder errechneten und gespeicherten Werten bekannter Lasten und Bestimmung der Abweichung der IST-Lage der Patientenliege (15) von der SOLL-Lage infolge Durchbiegung,
- Kompensation der Abweichung von der SOLL-Lage durch Verstellen der Höhe mindestens einer der Hubspindeln (27, 29, 31).

4. Verfahren nach Anspruch 3, **dadurch gekennzeichnet, dass** beim Verschieben der Patientenliege (15) auf einer weiteren, in einem Winkel zur ersten Verschieberichtung verlaufenden zweiten Strecke die Änderung der Lasten auf mindestens zwei Säulen gemessen wird und ein Vergleich der gemessenen Lasten mit zuvor errechneten oder ausgemessenen und gespeicherten Werten bekannter Lasten erfolgt, die Abweichung der IST-Lage der Patientenliege von der SOLL-Lage infolge Durchbiegung bestimmt wird und zur Kompensation die Höhen mindestens zweier Säulen (37) geändert werden.

5. Vorrichtung zum Heben und Senken sowie Drehen und horizontalen Verfahren einer Patientenliege (15) an einem Patiententisch (17) für eine Bestrahlungsanlage (7), umfassend einen Drehteller (23), eine Hubvorrichtung (21), eine Linearverschiebevorrichtung (3,5) für die Patientenliege (15) auf der Hubeinrichtung (21), eine Schwenkvorrichtung am freien Ende der Linearverschiebevorrichtung (3,5), an der die Patientenliege (15) angelenkt drehbar ist, **dadurch gekennzeichnet, dass** die Hubeinrichtung (21) mindestens drei Hubspindeln (27,29,31) umfasst, von denen mindestens eine der Hubspindeln (27) zum Messen der auf ihr wirkenden Last auf einer Lastzelle (41) abestützt ist, sowie eine Steuerung mit einem Rechner zum Vergleichen der IST- und SOLL-Werte der Position der Patientenliege (15) anhand der gemessenen Last und zur Steuerung der Antriebe der Elemente zum Korrigieren der errechneten Abweichungen vom SOLL-Wert.

6. Vorrichtung nach Anspruch 5, **dadurch gekennzeichnet, dass** zum Korrigieren der errechneten Abweichungen in der Steuerung aus Messreihen aufgefundene Messwerte gespeichert sind.

## Claims

1. A method for measuring and compensating for the deflection of the components of a patient table (17) on which the patient (13) lying on a patient support (15) can be moved into the focus of the energy beam (11) of a radiation system (7), said deflection being caused by the load of the patient, **characterized by** the following method steps:
- measuring the changes in the load, when moving the patient table (17) linearly, on at least one of three lifting spindles (27, 29, 31) that carry the patient support (15) and correcting/compensating for the deflection of the components of the patient table (17) by adjusting the height of at least one of the lifting spindles (27, 29, 31) in order to compensate for the deflection, said deflection being caused by the respective weight distribution of the patient.

2. The method according to claim 1, **characterized in that** correction/compensation is performed on the basis of measured values that have been determined by means of a series of measurements and stored or have been calculated and stored.

3. The method according to claim 1, **characterized in that** correction/compensation is performed by
- displacing the patient support (15) along a first stretch and measuring the change in the load as a function of the covered distance on at least one of three lifting spindles (27, 29, 31) that carry the patient support (15),
- comparing the load value(s) measured on at least one of the lifting spindles (27, 29, 31) with values of known loads that have been previously calculated or measured, determined by means of a series of measurements or calculated and stored, and determining the deviation of the actual position of the patient support (15) from the desired position as a result of deflection,
- compensating for the deviation from the desired position by adjusting the height of at least one of the lifting spindles (27, 29, 31).

4. The method according to claim 3, **characterized in that**, when displacing the patient support (15) along a further, second stretch extending at an angle relative to the first direction of displacement, the change in the loads is measured on at least two columns and the measured loads are compared with previously calculated or measured and stored values of known loads, the deviation of the actual position of the patient support from the desired position as a result of deflection is determined, and the heights of at least two columns (37) are changed for the purpose of compensation.

5. A device for lifting, lowering, rotating and horizontally moving a patient support (15) on a patient table (17) for a radiation system (7), comprising a rotary table (23), a lifting device (21), a linear-displacement device (3, 5) for the patient support (15) on the lifting device (21), a sluing device at the free end of the linear-displacement device (3, 5) where the patient support (15) is hinged, **characterized in that** the lifting device (21) comprises at least three lifting spindles (27, 29, 31), at least one of said lifting spindles (27) being supported, for the purpose of measuring the load acting on it, on a load cell (41), as well as a controlling means with a computer for comparing the actual values of the position of the patient support (15) with the desired values thereof on the basis of the measured load and for controlling the drives of the elements for correcting the calculated deviations from the desired value.

6. The device according to claim 5, **characterized in that** for correcting the calculated deviations, measured values obtained from series of measurements are stored in the controlling means.

## Revendications

1. Procédé de mesure et de compensation des flexions, provoquées par la charge d'un patient, des composants d'une table pour patient (17) sur laquelle le patient (13), allongé sur une couchette de patient (15), peut être positionné dans le foyer du faisceau d'énergie (11) d'une unité d'irradiation (7), **caractérisé par** les étapes de procédé suivantes :
- mesure des variations de la charge lors du déplacement linéaire de la table pour patient (17) au niveau d'au moins trois broches de levage (27, 29, 31) portant la couchette de patient (15) et correction/compensation des flexions des composants de la table pour patient (17) suscitées par la répartition pondérale respective du patient par le réglage de la hauteur d'au moins une des broches de levage (27, 29, 31) pour la compensation des flexions.

2. Procédé selon la revendication 1, **caractérisé en ce que** la correction/compensation s'effectue à l'aide de valeurs de mesure enregistrées, déterminées par une série de mesures, ou calculées et enregistrées.

3. Procédé selon la revendication 1, **caractérisé en ce que** la correction/compensation s'effectue par
- le coulissement de la couchette de patient (15) le long d'un premier trajet et la mesure de la variation de la charge en fonction de la distance parcourue au niveau d'au moins une de trois broches de levage (27, 29, 31) portant la couchette de patient (15),
- comparaison de la 1 des valeur(s) de charge mesurées au niveau d'au moins une des broches de levage (27, 29, 31) avec des valeurs de charges connues calculées ou mesurées précédemment, déterminées par une série de mesures ou calculées et enregistrées, et définition de la différence entre la position EFFECTIVE de la couchette de patient (15) et la position THEORIQUE du fait de la flexion,
- compensation de la différence par rapport à la position THEORIQUE par le réglage de la hauteur d'au moins une des broches de levage (27, 29, 31).

4. Procédé selon la revendication 3, **caractérisé en ce que**, lors du coulissement de la couchette de patient (15) sur un autre deuxième trajet faisant un angle par rapport à la première direction de coulissement, la variation des charges est mesurée sur au moins deux colonnes, et une comparaison des charges mesurées avec des valeurs de charges connues calculées ou mesurées précédemment et enregistrées est effectuée, la différence entre la position EFFECTIVE de la couchette de patient et la position THEORIQUE du fait de la flexion est définie et, pour la compensation, les hauteurs d'au moins deux colonnes (37) sont modifiées.

5. Dispositif de relèvement et d'abaissement ainsi que de rotation et de déplacement horizontal d'une couchette de patient (15) sur une table pour patient (17) pour une unité d'irradiation (7), comprenant un plateau rotatif (23), un dispositif de levage (21), un dispositif de coulissement linéaire (3, 5) pour la couchette de patient (15) sur le dispositif de levage (21), un dispositif de pivotement à l'extrémité libre du dispositif de coulissement linéaire (3, 5) au niveau duquel la couchette de patient (15) peut tourner de façon articulée, **caractérisé en ce que** le dispositif de levage (21) comprend au moins trois broches de levage (27, 29, 31), dont au moins une des broches de levage (27) est appuyée sur une cellule de charge (41) pour la mesure de la charge qui agit sur elle, ainsi qu'une commande avec un calculateur pour la comparaison des valeurs EFFECTIVES et THEORIQUES de la position de la couchette de patient (15) à l'aide de la charge mesurée et pour la commande des entraînements des éléments pour la correction des différences calculées par rapport à la valeur THEORIQUE.

6. Dispositif selon la revendication 5, **caractérisé en ce que**, pour la correction des différences calculées, des valeurs de mesures trouvées dans la commande et provenant des séries de mesures sont enregistrées.
